(19) Europäisches Patentamt
European Patent Office
Office européen des brevets

(11) Veröffentlichungsnummer: **0 318 767**
**A2**

(12) **EUROPÄISCHE PATENTANMELDUNG**

(21) Anmeldenummer: **88119145.6**

(22) Anmeldetag: **17.11.88**

(51) Int. Cl.⁴: **C07D 501/04 , C07D 499/32 , C07D 499/00 , C07D 471/04 , C07D 498/04 , C07D 513/04 , C07D 487/04 , //(C07D471/04, 221:00,205:00),(C07D498/04, 265:00,205:00),(C07D513/04, 279:00,205:00),(C07D487/04, 209:00,205:00)**

Claims for the following Contracting States: ES + GR.

(30) Priorität: **24.11.87 CH 4570/87**
**25.08.88 CH 3165/88**

(43) Veröffentlichungstag der Anmeldung:
**07.06.89 Patentblatt 89/23**

(84) Benannte Vertragsstaaten:
**AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Furlenmeier, André, Dr.**
**Wettsteinallee 119**
**CH-4058 Basel(CH)**
Erfinder: **Hubschwerlen, Christian, Dr.**
**Rue de la Gendarmerie**
**F-68200 Durmenach(FR)**
Erfinder: **Montavon, Marc, Dr.**
**Realpstrasse 72**
**CH-4054 Basel(CH)**

(74) Vertreter: **Lederer, Franz, Dr. et al**
**Patentanwält Dr. Franz Lederer Lucile Grahnstrasse 22**
**D-8000 München 80(DE)**

(54) **Unter physiologischen Bedingungen spaltbare Ester von pharmakologisch wirksamen Carbonsäuren.**

(57) Es werden neue, unter physiologischen Bedingungen spaltbare Ester von pharmakologisch wirksamen Carbonsäuren, insbesondere von antibiotisch wirksamen Carbonsäuren aus dem Gebiet der $\beta$-Lactam-Antibiotika beschrieben, welche dadurch gekennzeichnet sind, dass die Alkohol-Komponente des Esters eine Gruppe der allgemeinen Formel

EP 0 318 767 A2

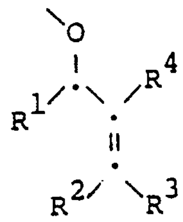

i

ist,

worin $R^1$ Wasserstoff oder niederes Alkyl, $R^2$ Wasserstoff, niederes Alkyl, niederes Halogenalkyl, niederes Alkenyl, niederes Alkoxycarbonyl, Aryl oder Heteroaryl oder zusammen mit $R^1$ niederes Alkylen, $R^3$ Wasserstoff, niederes Alkyl oder niederes Alkoxycarbonyl, $R^4$ die Gruppe $-COOR^5$, $-COR^6$, $-SO_2-R^6$, $-CONR^7R^8$ oder $-PO(OR^9)_2$, $R^5$ einen gesättigten oder ungesättigten Kohlenwasserstoffrest mit bis zu 12 Kohlenstoffatomen, in welchem bis zu zwei Methylengruppen durch Sauerstoffatome ersetzt sein können, Aryl oder Aryl-niederes Alkyl, $R^6$ niederes Alkyl oder Aryl, $R^7$ und $R^8$ je Wasserstoff oder niederes Alkyl oder zusammen niederes Alkylen, in welchem eine Methylengruppe durch ein Sauerstoff- oder Schwefelatom oder durch eine Imino- oder niedere Alkyliminogruppe ersetzt sein kann, und $R^9$ niederes Alkyl bedeuten.

Diese Ester können, sofern ein basischer Substituent vorhanden ist, auch in Form von pharmazeutisch annehmbaren Säureadditionssalzen vorliegen.

# Unter physiologischen Bedingungen spaltbare Ester von pharmakologisch wirksamen Carbonsäuren

Die vorliegende Erfindung betrifft unter physiologischen Bedingungen spaltbare Ester von pharmakologisch wirksamen Carbonsäuren insbesondere von antibiotisch wirksamen Carbonsäuren aus dem Gebiet der $\beta$-Lactam-Antibiotika, welche dadurch gekennzeichnet sind, dass die Alkohol-Komponente des Esters eine Gruppe der allgemeinen Formel

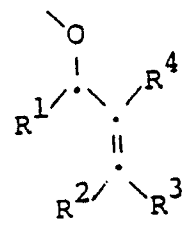

i

ist,

worin $R^1$ Wasserstoff oder niederes Alkyl, $R^2$ Wasserstoff niederes Alkyl, niederes Halogenalkyl, niederes Alkenyl, niederes Alkoxycarbonyl, Aryl oder Heteroaryl oder zusammen mit $R^1$ niederes Alkylen, $R^3$ Wasserstoff, niederes Alkyl oder niederes Alkoxycarbonyl, $R^4$ die Gruppe $-COOR^5$, $-COR^6$, $-SO_2-R^6$ - $CONR^7R^8$ oder $-PO(OR^9)_2$, $R^5$ einen gesättigten oder ungesattigten Kohlenwasserstoffrest mit bis zu 12 Kohlenstoffatomen, in welchem bis zu zwei Methylengruppen durch Sauerstoffatome ersetzt sein können, Aryl oder Aryl-niederes Alkyl, $R^6$ niederes Alkyl oder Aryl, $R^7$ und $R^8$ je Wasserstoff oder niederes Alkyl oder zusammen niederes Alkylen, in welchem eine Methylengruppe durch ein Sauerstoff- oder Schwefelatom oder durch eine Imino- oder niedere Alkyliminogruppe ersetzt sein kann, und $R^9$ niederes Alkyl bedeuten,

und, sofern ein basischer Substituent vorhanden ist pharmazeutisch annehmbare Säureadditionssalze davon.

Die obigen Ester sind neu und besitzen wertvolle pharmakologische Eigenschaften. Sie werden unter physiologischen Bedingungen zu den freien, pharmakologisch wirksamen Carbonsäuren gespalten, welche dann ihre therapeutischen Wirkungen entfalten. Sie können demnach zur Verhütung oder Bekämpfung von Krankheiten verwendet werden, wobei sie sich für die enterale, insbesondere für die perorale Applikation eignen.

Gegenstand der vorliegenden Erfindung sind: Die obigen unter physiologischen Bedingungen spaltbaren Ester von pharmakologisch wirksamen Carbonsäuren, insbesondere von antibiotisch wirksamen Carbonsäuren aus dem Gebiet der $\beta$-Lactam-Antibiotika als solche und zur Anwendung als theraPeutische Wirkstoffe; ein Verfahren und Zwischenprodukte zu deren Herstellung; diese enthaltende Arzneimittel und ein Verfahren zur Herstellung derartiger Arzneimittel; die Verwendung dieser Ester zur Verhütung oder Bekämpfung von Krankheiten und zur Herstellung von Arzneimitteln, insbesondere von antibiotisch wirksamen Mitteln; sowie die Verwendung gewisser Ausgangsstoffe bei der Veresterung von pharmakologisch wirksamen Carbonsäuren, insbesondere von antibiotisch wirksamen Carbonsäuren aus dem Gebiet der $\beta$-Lactam-Antibiotika.

Der für die Definition der Carbonsäure-Komponente der erfindungsgemässen Ester verwendete Ausdruck "pharmakologisch wirksame Carbonsäure" ist im weitesten Sinne zu verstehen und umfasst sämtliche, pharmakologisch wirksamen Verbindungen, welche als Strukturelement eine freie Carbonsäuregruppe enthalten.

Auch der Ausdruck "antibiotisch wirksame Carbonsäure aus dem Gebiet der $\beta$-Lactam-Antibiotika" ist im weitesten Sinne zu verstehen. Er umfasst sämtliche, antibiotisch wirksamen Verbindungen, welche als Strukturelemente einen $\beta$-Lactamring und eine freie Carbonsäuregruppe enthalten. Bevorzugt werden bicyclische $\beta$-Lactam-Antibiotika, in welchen der $\beta$-Lactamring Bestandteil des Bicyclus ist und sich die freie Carbonsäuregruppe in $\alpha$-Stellung zum $\beta$-Lactamstickstoffatom befindet. Die Carbonsäure-Komponente in den erfindungsgemässen Estern ist demnach vorzugsweise ein Penam-3-carbonsäure-, Penem-3-carbonsäure-, 1-Carbapenem-3-carbonsäure-, 3-Cephem-4-carbonsäure-, 1-Oxa-3-cephem-4-carbonsäure-, 1-Dethia-2-oxa-3-cephem-, 1-Dethia-2-thia-3-cephem- oder ein 1-Carba-3-cephem-4-carbonsäurerest.

In einer besonders bevorzugten Ausführungsform ist die Carbonsäure-Komponente der erfindungsgemässen Ester ein pharmazeutisch annehmbarer 3-Cephem-4-carbonsäurerest. In einer ganz besonders

bevorzugten Ausführungsform betrifft die vorliegende Erfindung Ester der allgemeinen Formel

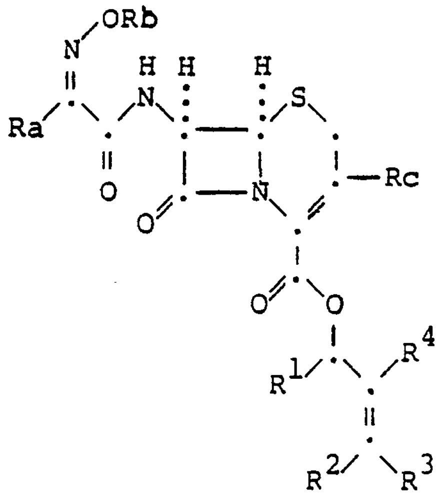

I

worin Ra Aryl oder Heteroaryl, Rb Wasserstoff, niederes Alkanoyl oder gegebenenfalls durch Carboxy, Halogen, $C_{3-6}$-Cyloalkyl oder Heteroaryl substituiertes niederes Alkyl oder $C_{3-6}$-Cycloalkyl, Rc Wasserstoff, Halogen, niederes Alkyl, niederes Alkenyl, niederes Alkoxy oder die Gruppe $-CH_2$-Rd oder $-CH_2$-S-Re, Rd niederes Alkanoyloxy, Carbamoyloxy, Azido oder eine über ein Stickstoffatom gebundene, N-haltige aromatische Gruppe und Re eine über ein Kohlenstoffatom gebundene heteroaromatische Gruppe bedeuten, und $R^1$, $R^2$ $R^3$ und $R^4$ obige Bedeutung besitzen.

Der Ausdruck "nieder" wird verwendet, um Reste und Verbindungen mit bis zu 7, vorzugsweise bis zu 4 Kohlenstoffatomen zu bezeichnen. Der Ausdruck "Alkyl" bezeichnet geradkettige oder verzweigte, gesättigte Kohlenwasserstoffreste, wie Methyl, Aethyl, Propyl, Isopropyl, Butyl, Isobutyl, s-Butyl, t-Butyl und 2-Aethyl-hexyl. Der Ausdruck "Halogenalkyl" bezeichnet eine durch Halogen mono-, di- oder trisubstituierte Alkylgruppe, wie Chlormethyl, Trichlormethyl und Trifluormethyl. Der Ausdruck "Alkoxy" bezeichnet eine über ein Sauerstoffatom gebundene Alkylgruppe. Der Ausdruck "Alkylen" bezeichnet geradkettige oder verzweigte, gesättigte Kohlenwasserstoffreste mit zwei freien Valenzen, wie Dimethylen, Trimethylen und Tetramethylen. Der Ausdruck "Alkenyl" bezeichnet geradkettige oder verzweigte Kohlenwasserstoffreste, welche mindestens eine olefinische Doppelbindung enthalten, wie Vinyl, 1-Propenyl und 2-Propenyl.

Der Ausdruck "gesättigter oder ungesättigter Kohlenwasserstoffrest mit bis zu 12 Kohlenstoffatomen, in welchem bis zu zwei Methylengruppen durch Sauerstoffatome ersetzt sein können," bezeichnet offenkettige und cyclische Gruppen und Kombinationen davon. Beispiele für offenkettige Reste, welche geradkettig oder verzweigt sein können, sind: Alkyl-, Alkenyl- und Alkoxyalkylgruppen, wie sie weiter oben definiert sind, und Alkoxyalkoxyalkyl- und Dialkoxyalkylgruppen, wie 2,3-Dimethoxypropyl. Beispiele für cyclische Gruppen und Kombinationen von cyclischen und offenkettigen Gruppen sind:
Gegebenenfalls durch Alkylgruppen substituierte Cycloalkyl-und Cyloalkylalkylgruppen, worin die cyclische Gruppe bis zu 7 Ringglieder besitzt und worin bis zu zwei Methylengruppen durch Sauerstoffatome ersetzt sein können, wie Tetrahydro-2H-pyran-4-yl, (Tetrahydro-2H-pyran-2-yl)methyl, Cyclohexyl und Cyclohexylmethyl.

Der Ausdruck "Aryl" bezeichnet carbocyclische aromatische Gruppen, vorzugsweise monocyclische Gruppen, d.h. Phenylgruppen. Diese Gruppen sind gegebenenfalls durch niederes Alkyl, niederes Alkoxy, Halogen, Trifluormethyl oder Cyano substituiert. Bevorzugt werden unsubstituierte oder monosubstituierte Gruppen, wie Phenyl.

Der Ausdruck "Heteroaryl" bezeichnet heterocyclische aromatische Gruppen, vorzugsweise 5- oder 6-gliedrige, monocyclische, aromatische Heterozyklen. Die 5-gliedrigen aromatischen Heterozyklen enthalten als Heteroringglieder vorzugsweise ein Sauerstoff- oder Schwefelatom oder eine Imino- oder niedere Alkyliminogruppe und gegebenenfalls noch ein oder zwei Stickstoffatome. Die 6-gliedrigen aromatischen Heterozyklen enthalten als Ringglieder vorzugsweise ein, zwei oder drei Stickstoffatome. Als Beispiele seien erwähnt:
Thienyl, Thiazolyl, Thiadiazolyl und Furyl. Diese Gruppen sind unsubstituiert oder durch niederes Alkyl, niederes Alkoxy, Halogen, Trifluormethyl, Nitro, Amino oder Cyano substituiert. Sie sind vorzugsweise unsubstituiert oder monosubstituiert. Als Beispiele seien erwähnt: 3-Thienyl, 2-Furyl, 2-Amino-4-thiazolyl und 2-Brom-5-furyl.

Der Ausdruck "eine über ein Stickstoffatom gebundene, N-haltige aromatische Gruppe" bezeichnet aromatische heterocyclische Gruppen, welche als Heteroringglieder bis zu 4 Stickstoffatome enthalten. Sie sind vorzugsweise 5- oder 6-gliedrig und können noch durch einen 5- oder 6-gliedrigen Cycloalkanring oder durch einen Benzolring anneliert sein. Sie sind vorzugsweise unsubstituiert oder durch niederes Alkyl oder Carbamoyl substituiert. 5-Methyl-2-tetrazolyl ist ein Beispiel für N-haltige heterocyclische Gruppen.

Der Ausdruck "eine über ein Kohlenstoffatom gebundene heteroaromatische Gruppe" bezeichnet vorzugsweise monocyclische, insbesondere 5- und 6-gliedrige, aromatische heterocyclische Gruppen, welche als Heteroatome vorzugsweise ein Sauerstoff- oder Schwefelatom und/oder 1-4 Stickstoffatome enthalten, und bicyclische, insbesondere 8- bis 10-gliedrige, aromatische heterocyclische Gruppen, welche als Heteroatome vorzugsweise ein Sauerstoff- oder Schwefelatom und/oder 1-5 Stickstoffatome enthalten. Diese Gruppen sind vorzugsweise unsubstituiert oder durch niederes Alkyl, niederes Alkoxy, niederes Alkandiyl, Halogen, Trifluormethyl, niederes Alkoxycarbonyl oder Carbamoyl substituiert.

Ra bedeutet vorzugsweise eine unsubstituierte oder durch Amino substituierte 5-gliedrige heteroaromatische Gruppe mit einem Schwefel- oder Sauerstoffatom und gegebenenfalls noch einem oder zwei Stickstoffatome als Heteroringglieder, insbesondere 2-Amino-4-thiazolyl, 5-Amino-1,2,4-thiadiazol-3-yl oder 2-Furyl. In einer ganz besonders bevorzugten Ausführungsform bedeutet Ra 2-Amino-4-thiazolyl.

Rb bedeutet vorzugsweise Wasserstoff, niederes Alkyl oder niederes Alkanoyl, insbesondere niederes Alkyl.

Rc bedeutet vorzugsweise niederes Alkyl, insbesondere Methyl, oder die Gruppe $-CH_2-Rd$, wobei Rd vorzugsweise Azido bedeutet.

$R^1$ bedeutet vorzugsweise Wasserstoff.

$R^2$ bedeutet vorzugsweise niederes Alkyl.

$R^3$ bedeutet vorzugsweise Wasserstoff.

$R^4$ bedeutet vorzugsweise die Gruppe $-COOR^5$, und $R^5$ bedeutet vorzugsweise niederes Alkyl, niederes Alkenyl oder niederes Alkoxyalkyl. In einer besonders bevorzugten Ausführungsform bedeutet $R^5$ niederes Alkyl oder niederes Alkoxyalkyl, insbesondere Isobutyl, Isopropyl oder 2-Aethoxyäthyl, wobei die Bedeutungsmöglichkeit Isobutyl ganz besonders bevorzugt wird.

Die nachfolgend aufgeführten Verbindungen sind ganz besonders bevorzugte, erfindungsgemässe Ester:

(E)-2-(Isobutoxycarbonyl)-2-pentenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat,
(E)-2-(Isobutoxycarbonyl)-2-butenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat,
(E)-2-[(2-Aethoxyäthoxy)carbonyl]-2-butenyl (6R,7R)-7-[(Z)-2-(2-amino-4-thiazolyl)-2-(methoxyimino)-acetamido]-3-(azidomethyl)-8-oxo-5-thia-1-azabicyclo[4.2.0]oct-2-en-2-carboxylat,
(E)-2-Aethoxycarbonyl)-2-butenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat,
(E)-2-[[(3-Methyl-2-butenyl)oxy]carbonyl]-2-butenyl (6R,7R)-7-[(Z)-2-(2-amino-4 thiazolyl)-2-(methoxyimino)-acetamido]-3-methyl-8-oxo-5-thia-1-azabicyclo[4.2.0]oct-2-en-2-carboxylat,
(E)-2-(Isopropoxycarbonyl)-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat,
(E)-2-(Isobutoxycarbonyl)-2-pentenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat,
(E)-2-(Aethoxycarbonyl)-2-pentenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat,
(E)-2-[(2-Aethoxyäthoxy)carbonyl]-2-butenyl (6R,7R)-7-[(Z)-2-(2-amino-4 thiazolyl)-2-(methoxyimino)-acetamido]-3-methyl-8-oxo-5-thia-1-azabicyclo[4.2.0]oct-2-en-2-carboxylat,
(E)-2-(Isobutoxycarbonyl)-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat,
(E)-2-(t-Butoxycarbonyl)-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat und
(E)-2-(Aethoxycarbonyl)-2-butenyl (6R,7R)-7-[(Z)-2-(2-amino-4-thiazolyl)-2-(methoxyimino)acetamido]-3-methyl-8-oxo-5-thia-azabicyclo[4.2.0]oct-2-en-2-carboxylat.

Die erfindungsgemässen Ester und, sofern sie eine basische Gruppe besitzen, ihre pharmazeutisch annehmbaren Säureadditionssalze können hergestellt werden, indem man eine pharmakologisch wirksame Carbonsäure mit einer Verbindung der allgemeinen Formel

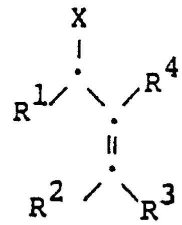

II

worin X Hydroxy oder eine Abgangsgruppe bedeutet, und $R^1$, $R^2$, $R^3$ und $R^4$ obige Bedeutung besitzen, verestert, oder

b) zur Herstellung eines Esters der obigen Formel I eine Verbindung der allgemeinen Formel

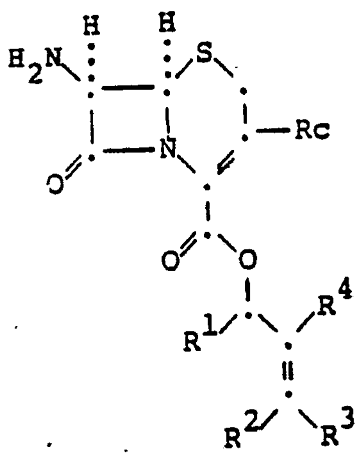

III

worin $R^1$, $R^2$, $R^3$, $R^4$ und Rc obige Bedeutung besitzen, mit einer Carbonsäure der allgemeinen Formel

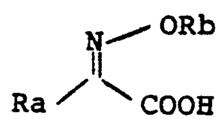

IV

worin Ra und Rb obige Bedeutung besitzen, oder einem reaktiven Derivat davon acyliert und

c) erwünschtenfalls den erhaltenen Ester in ein pharmazeutisch annehmbares Säureadditionssalz überführt.

Die Veresterung gemäss Verfahrensvariante a) kann nach an sich bekannten und jedem Fachmann geläufigen Methoden durchgeführt werden. Man kann beispielsweise ein Salz, insbesondere ein Alkalimetallsalz, wie das Natrium- oder Kaliumsalz, einer pharmakologisch wirksamen Carbonsäure mit einer Verbindung der Formel II umsetzen, worin X eine Abgangsgruppe bedeutet. Geeignete Abgangsgruppen sind z.B. Halogenatome, wie Brom und Jod, und niedere Alkyl- und Arylsulfonyloxygruppen, wie Methylsulfonyloxy und p-Toluol sulfonyloxy. Geeignete Lösungsmittel sind beispielsweise Dimethylformamid und Dimethylacetamid. Die Reaktion wird vorzugsweise in einem Temperaturbereich von etwa -30 °C bis etwa Raumtemperatur vorzugsweise bei etwa 0 °C durchgeführt.

Es ist aber auch möglich, die freie, pharmnakologisch wirksame Carbonsäure in Gegenwart eines Kondensationsmittels mit einer Verbindung der Formel II umzusetzen, worin X eine Hydroxygruppe bedeutet. Ein geeignetes Kondensationsmittel ist beispielsweise Diäthylazodicarboxylat/Triphenylphosphin. Geeignete Lösungsmittel sind beispielsweise Dimethylformamid und Dimethylacetamid. Die Reaktion wird vorzugsweise in einem Temperaturbereich von -30 °C bis etwa Raumtemperatur, vorzugsweise bei etwa 0 °C durchgeführt.

Die Acylierung gemass Verfahrensvariante (b) kann ebenfalls nach an sich bekannten und jedem Fachmann geläufigen Methoden durchgeführt werden. Man kann beispielsweise die Verbindung der Formel

III mit der freien Carbonsäure der Formel IV oder einem Salz davon mit einer Base acylieren. wobei man in diesem Fall in Gegenwart eines geeigneten Kondensationsmittels arbeitet. Geeignete Kondensationsmittel sind beispielsweise N,N′-disubstituierte Carbodiimide, wie N,N′-Dicyclohexylcarbodiimid, welche vorzugsweise zusammen mit N-Hydroxybenzotriazol oder N-Hydroxysuccinimid verwendet werden. 2-Halogenpyridiniumsalze, wie 2-Chlor-1-methylpyridiniumchlorid, Phosphoroxychlorid, Thionylchlorid und Oxalylchlorid.

Es ist aber auch möglich, die Carbonsäure der Formel IV in Form eines reaktiven Derivates einzusetzen. Als reaktionsfähige Derivate kommen insbesondere Säurechloride, Säureanhydride, gemischte Anhydride (beispielsweise Anhydride mit Trifluoressigsäure, Benzolsulfonsäure, Mesitylensulfonsäure, p-Toluolsulfonsäure und p-Chlorsulfonsäure) und aktive Thiolester, beispielsweise S-(2-Benzothiazolyl)thioester in Frage.

Die Acylierung kann gegebenenfalls in Gegenwart eines säurebindenden Mittels durchgeführt werden, wobei als säurebindende Mittel insbesondere Natriumhydrogencarbonat, Kaliumcarbonat, Triäthylamin, Pyridin oder N-Methylmorpholin in Frage kommen. Geeignete Lösungsmittel sind beispielsweise cyclische Aether, wie Tetrahydrofuran und Dioxan, halogenierte niedere Kohlenwasserstoffe, wie Chloroform und Methylenchlorid, Dimethylformamid, Dimethylacetamid, Acetonitril, Aceton und Wasser, sowie Mischungen davon. Die Reaktionstemperatur kann in einem weiten Bereich variieren und liegt in der Regel zwischen -50° C und 50° C, vorzugsweise zwischen etwa -10° C und 30° C.

Die als Ausgangsstoffe verwendeten Verbindungen der Formel III sind neu und ebenfalls Gegenstand der vorliegenden Erfindung. Sie können durch Veresterung der entsprechenden 7-Amino-3-cephem-4-carbonsäuren in Analogie zu Verfahrensvariante a) hergestellt werden.

Die Herstellung von Säureadditionssalzen der erfindungsgemässen Ester, welche basisch substituiert sind, kann ebenfalls nach an sich bekannten und jedem Fachmann geläufigen Methoden erfolgen. Es kommen dabei sowohl Salze mit pharmazeutisch annehmbaren anorganischen als auch Salze mit pharmazeutisch annehmbaren organischen Säuren in Betracht. Beispiele von Säureadditionssalzen der erfindungsgemässen Ester sind Salze mit Mineralsäuren, beispielsweise Halogenwasserstoffsäuren, wie Salzsäure, Bromwasserstoff und Jodwasserstoff, Schwefelsäure, Salpetersäure, Phosphorsäure und dergleichen, Salze mit organischen Sulfonsäuren, beispielsweise mit Alkyl- und Arylsulfonsäuren, wie Aethansulfonsäure, p-Toluolsulfonsäure, Benzolsulfonsäure und dergleichen, sowie Salze mit organischen Carbonsäuren, bei spielsweise mit Essigsäure, Weinsäure, Maleinsäure, Zitronensäure, Benzoesäure, Salicylsäure, Ascorbinsäure und dergleichen.

Die erfindungsgemässen Ester können auch hydratisiert sein. Derartige Hydrate werden zumeist automatisch im Zuge des Herstellungsverfahrens oder als Folge hygroskopischer Eigenschaften eines zunächst wasserfreien Produktes erhalten. Zur gezielten Herstellung eines Hydrates kann ein ganz oder teilweise wasserfreies Produkt einer feuchten Atmosphäre ausgesetzt werden.

Die als Ausgangsstoffe verwendeten pharmakologisch wirksamen Carbonsäuren, insbesondere antibiotisch wirksamen Carbonsäuren aus dem Gebiet der β-Lactam-Antibiotika gehören an sich bekannten Stoffklassen an. Spezifisch nicht vorbeschriebene Vertreter dieser Stoffklassen können in Analogie zu den bekannten Vertretern nach an sich bekannten Methoden hergestellt werden.

Die als Ausgangsstoffe verwendeten Verbindungen der Formel II gehören ebenfalls an sich bekannten Stoffklassen an. Spezifisch nicht vorbeschriebene Vertreter dieser Stoffklasse können in Analogie zu den bekannten Vertretern nach an sich bekannten Methoden hergestellt werden. Es wird in diesem Zusammenhang auf die nachfolgend aufgeführten Publikationen verwiesen: Deutsche Offenlegungsschrift Nr. 2155113 (publiziert am 10. Mai 1972); H.M.R. Hoffmann und J. Rabe, Angew. Chem. 95, 795-796 (1983): F. Ameer et al., J. Chem. Soc. Perkin Trans. 1, 1983, 2293-2295: und P. Knochel und J.F. Normant, J. Organamet. Chemistry 309, 1-23 (1986).

Wie bereits eingangs erwähnt besitzen die den erfindungsgemässen Estern entsprechenden Carbonsäuren wertvolle Pharmakologische Eigenschaften. Sie eignen sich ganz besonders zur enteralen, insbesondere peroralen Behandlung von Krankheiten.

Die perorale antimikrobielle Wirksamkeit der erfindungsgemässen Ester von antibiotisch wirksamen Carbonsäuren kann beispielsweise im nachfolgend beschriebenen Tierversuch nachgewiesen werden:

Man verursacht eine experimentelle Septikämie in 16-20 g schweren Albinomäusen, indem man den Versuchstieren eine Probe einer verdünnten Kultur des Testorganismus, Escherichia coli 25922, welche über Nacht gewachsen ist. intraperitoneal injiziert. Die verabreichte Probe wird dabei so bemessen, dass die verabreichte Dosis etwa 10-20 mal grösser ist, als die Dosis, welche benötigt wird um 50% der unbehandelten Versuchstiere innerhalb von 48 Stunden zu töten. Die Bakterien wurden als Suspension in Kulturbrühe injiziert. Sowohl für die Kontrolle als auch für die Behandlung wurden für jede getestete Dosis Gruppen zu je 5 Versuchstieren verwendet. Die erfindungsgemässen Ester wurden oral verabreicht, und zwar als Lösung in Tween 80 (4%), wobei die Konzentrationen so gewählt wurden, dass jedes Versuchstier

500 µl erhielt. Die Behandlungen erfolgten 1 und 3 Stunden nach der Infektion. Die $ED_{50}$ ist diejenige Dosis, welche 50% der Versuchstiere schützt, und wurde ausgehend von der Ueberlebensrate nach 4 Tagen nach der Infektion mittels der Probitmethode berechnet. In der nachfolgenden Tabelle sind die $ED_{50}$-Werte in mg/kg p.o. für Vertreter der erfindungsgemassen Stoffklasse angegeben. Die Tabelle enthält ausserdem Angaben über die akute Toxizität der erfindungsgemässen Ester bei einmaliger oraler Verabreichung an Mäusen.

Tabelle

| Erfindungsgemässe Ester | $ED_{50}$ in mg/kg p.o. | $DL_{50}$ in mg/kg p.o. |
|---|---|---|
| Verbindung A | 0,28 | >5000 |
| Verbindung B | 1,4 | |
| Verbindung C | 0,89 | >5000 |

Verbindung A:
(E)-2-(Isobutoxycarbonyl)-2-pentenyl (6R,7R)-7-[(Z)-2-(2-amino-4-thiazolyl)-2-(methoxyimino)acetamido]-3-(azidomethyl)-8-oxo-5--thia-1-azabicyclo[4.2.0]oct-2-en-2-carboxylat

Verbindung B:
(E)-2-(Isobutoxycarbonyl)-2-pentenyl (6R,7R)-7-[(Z)-2-(2-amino-4-thiazolyl)-2-(methoxyimino)acetamido]-3-methyl-8-oxo-5-thia-1--azabicyclo[4.2.0]oct-2-en-2-carboxylat

Verbindung C:
(E)-2-(Aethoxycarbonyl)-2-butenyl (6R,7R)-7-[(Z)-2-(2-amino-4-thiazolyl)-2-(methoxyimino)acetamido]-3-methyl-8-oxo-5-thia-azabicyclo[4.2.0]oct-2-en-2-carboxylat.

Die erfindungsgemässen Produkte können als Heilmittel, z.B. in Form pharmazeutischer Präparate zur enteralen Applikation, Verwendung finden. Die erfindungsgemässen Produkte können beispielsweise peroral, z.B. in Form von Tabletten, Lacktabletten, Dragées, Hart- und Weichgelatinekapseln, Lösungen, Emulsionen oder Suspensionen, oder rektal, z.B. in Form von Suppositorien, verabreicht werden.

Die Herstellung der pharmazeutischen Präparate kann in jedem Fachmann geläufiger Weise dadurch erfolgen, dass man die erfindungsgemässen Stoffe, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als solche Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Träger beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser Polyole, Saccharose, Invertzucker und Glucose. Für Suppositorien eignen sich als Trägermaterialien beispielsweise natürliche oder gehärtete Oele, Wachse, Fette und halbflüssige oder flüssige Polyole.

Als pharmazeutische Hilfsstoffe kommen die üblichen Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel und Antioxydantien in Frage.

Die erfindungsgemässen Ester und ihre pharmazeutisch annehmbaren Säureadditionssalzsalze und Hydrate kommen für die enterale, insbesondere für die perorale Applikation in Betracht.

Die pharmazeutischen Präparate können die erfindungsgemässen Ester, je nach Natur der dem Ester entsprechenden pharmakologisch wirksamen Carbonsäure, in Mengen von etwa 25-2000 mg, vorzugsweise 100-1000 mg, pro Einzeldosierungsform enthalten. Für die Verhütung und Bekämpfung von Infektionskrankheiten kommt für den Erwachsenen eine tägliche Dosis von etwa 0,05 g bis etwa 4 g, insbesondere etwa 0,5 g bis etwa 2 g in Betracht. Diese Dosis kann als Einzeldosis oder über mehrere Dosen verteilt verabreicht werden.

Die nachfolgenden Beispiele sollen die vorliegende Erfindung näher erläutern, ihren Umfang jedoch in keiner Weise beschränken. Sämtliche Temperaturen sind in Celsiusgraden angegeben.

## Beispiel 1

a) 1 g (6R,7R)-7-[(Z)-2-(2-Aminc-4-thiazolyl) -2-(methoxyimino)acetamido]-3-methyl-8-oxo-5 thia -1-azabicyclo[4.2.0]-oct-2-en-2-carbonsäure Natriumsalz wird in 20 ml Dimethylformamid gelöst, auf 0° abgekühlt und mit 0,7 g Brommethylacrylsäureäthylester versetzt. Nach 2 Stunden gibt man 80 ml Wasser dazu und extrahiert mit 200 ml Essigester. Die organische Phase wird dreimal mit je 50 ml Wasser gewaschen, über Magnesiumsulfat getrocknet und auf ein Volumen von 20 ml eingeengt. Unter Rühren gibt man 100 ml Aether dazu, nutscht das ausgefallene Produkt ab und trocknet es im Vakuum bei Raumtemperatur. Man erhält 2-(Aethoxycarbonyl)allyl (6R,7R)-7-[(Z)-2-(2-amino-4-thiazolyl) -2-(methoxyimino)-acetamido]-3-methyl-8-oxo-5-thia -1-azabicyclo[4.2.0]oct-2-en-2-carboxylat als weissen Festkörper (aus Essigester/Aether).

| Elementaranalyse für $C_{20}H_{23}N_5O_7S_2$ (509,552): | | | | |
|---|---|---|---|---|
| | C | H | N | S |
| Ber. | 47,14 | 4,55 | 13,74 | 12,58 |
| Gef. | 46,82 | 4,64 | 13,50 | 12,38 |

In analoger Weise erhält man:

b) 2-(Aethoxycarbonyl)allyl (6R,7R)-7-[(Z)-2-(2-amino-4-thiazolyl)-2-(methoxyimino)acetamido]-3-[(5-methyl-2H-tetrazol-2-yl)methyl]-8-oxo-5-thia-1-azabicyclo[4.2.0]oct-2-en-2-carboxylat als weissen Festkörper (aus Essigester/Aether).

| Elementaranalyse für $C_{22}H_{25}N_9O_7S_2$ (591,62): | | | | |
|---|---|---|---|---|
| | C | H | N | S |
| Ber. | 44,66 | 4,26 | 21,31 | 10,84 |
| Gef. | 44,39 | 4,45 | 21,05 | 10,79 |

c) 2-(Aethoxycarbonyl)allyl (6R,7R)-3-[(carbamoyloxy)methyl]-7-[(Z)-2-(2-furyl)-2-(methoxyimno)-acetamido]-8-oxo-5-thia-1-azabicyclo[4.2.0]oct-2-en-2-carboxylat als weissen Festkörper (aus Essigester/i-Propyläther).

| Elementaranalyse für $C_{22}H_{24}N_4O_{10}S$ (536,521): | | | | |
|---|---|---|---|---|
| | C | H | N | S |
| Ber. | 49,25 | 4,51 | 10,44 | 5,98 |
| Gef. | 49,46 | 4,80 | 10,24 | 5,84 |

## Beispiel 2

a) 2 g (6R,7R)-7-[(Z)-2-(2-Amino-4-thiazolyl) -2-(methoxyimino)acetamido]-3-methyl-8-oxo-5-thia 1-azabicyclo[4.2.0]oct-2-en-2-carbonsäure-Natriumsalz werden in 40 ml Dimethylformamid gelöst, auf 0° abgekühlt und mit 1,58 g α-Brommethylacrylsäure-t-butylester versetzt. Nach 5 Stunden gibt man 200 ml Wasser dazu und extrahiert mit 350 ml Essigester. Die organische Phase wird dreimal mit je 100 ml Wasser gewaschen, über Magnesiumsulfat getrocknet und auf ein Volumen von 20 ml eingeengt. Unter Rühren werden 250 ml Isopropyläther dazugegeben. Das ausgefallene Produkt wird abgenutscht und im Vakuum bei Raumtemperatur getrocknet. Man erhält 2-(t-Butoxycarbonyl)allyl (6R,7R)-7-[(Z)-2-(2-amino-4-thiazolyl) -2-(methoxyimino)acetamido]-3-methyl-8-oxo-5-thia -1-azabicyclo[4.2.0]oct-2-en-2-carboxylat als weissen Festkörper (aus Essigester/i-Propyläther).

| Elementaranalyse für $C_{22}H_{27}N_5O_7S_2$ (537,61): | | | | |
|---|---|---|---|---|
| | C | H | N | S |
| Ber. | 49,15 | 5,06 | 13,03 | 11,93 |
| Gef. | 48,78 | 5,14 | 13,03 | 12,15 |

In analoger Weise erhält man:

b) 2-(t-Butoxycarbonyl)allyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat als beigen Festkörper (aus Essigester/i-Propyläther).

| Elementaranalyse für $C_{22}H_{26}N_8O_7S_2$ (578,63) + 0,15 Mol Essigester: | | | | |
|---|---|---|---|---|
| | C | H | N | S |
| Ber. | 45,67 | 4,52 | 19,36 | 11,08 |
| Gef. | 45,87 | 4,63 | 18,93 | 10,83 |

c) 2-(t-Butoxycarbonyl)allyl (6R,7R)-7-[(Z)-2-(2-amino-4-thiazolyl)-2-(methoxyimino)acetamido]-3-[(5-methyl-2H-tetrazol-2-yl)methyl]-8-oxo-5-thia-1-azabicyclo[4.2.0]oct-2-en-2-carboxylat als weissen Festkörper (aus Essigester/i-Propyläther).

| Elementaranalyse für $C_{24}H_{29}N_9O_7S_2$ (619,683): | | | | |
|---|---|---|---|---|
| | C | H | N | S |
| Ber. | 46,52 | 4,72 | 20,39 | 10,35 |
| Gef. | 46,33 | 4,84 | 20,12 | 10,60 |

d) 2-(t-Butoxycarbonyl)allyl (6R,7R)-3-[(carbamoyloxy)methyl]-7-[(Z)-2-(2-furyl)-2-(methoxyimino)-acetamido]-8-oxo-5-thia-1-azabicyclo[4.2.0]oct-2-en-2-carboxylat als weissen Festkörper (aus Essigester/Petroläther h.s.).

| Elementaranalyse für $C_{24}H_{28}N_4O_{10}S$ (564,576) + 0,25 Mol Essigester: | | | | |
|---|---|---|---|---|
| | C | H | N | S |
| Ber. | 51,06 | 5,00 | 9,92 | 5,68 |
| Gef. | 51,19 | 5,16 | 9,55 | 5,47 |

## Beispiel 3

Man behandelt eine Lösung von 2,5 mMol eines Alkalimetallsalzes einer antibiotisch wirksamen Carbonsäure aus dem Gebiet der β-Lactam-Antibiotika in 20 ml trockenem Dimethylformamid unter Kühlen mit Eiswasser mit einer Lösung von 2,7 mMol eines entsprechenden Halogenides der Formel II in 2 ml trockenem Dimethylformamid, entfernt das Kühlbad und rührt die erhaltene Lösung über Nacht bei etwa 0°C. Die Lösung wird dann mit 100 ml Essigester verdünnt und dreimal mit je 50 ml Wasser und einmal mit 50 ml Kochsalzlösung extrahiert. Die organische Phase wird über Magnesiumsulfat getrocknet, filtriert und eingedampft. Der Rückstand wird an einer kurzen Kieselgelsäule unter Eluieren mit Essigester/Hexan (8:2) rasch chromatographiert. Die relevanten Fraktionen werden vereinigt und eingedampft. Eine Lösung des Rückstandes in 2 ml Methylenchlorid wird dann tropfenweise zu 300 ml eines Gemisches aus Essigester und Hexan (1:1) gegeben. Die erhaltenen Kristalle werden abfiltriert und unter vermindertem Druck getrocknet.

Die nachfolgend aufgeführten Verbindungen wurden auf diese Weise hergestellt:

a) (E)-2-(Aethoxycarbonyl)-2-butenyl (6R,7R)-7-[(Z)-2-(2-amino-4-thiazolyl)-2-(methoxyimino)-acetamido]-3-methyl-8-oxo-5-thia-azabicyclo[4.2.0]oct-2-en-2-carboxylat als farblosen Festkörper (aus Methylenchlorid/Aether).

| Elementaranalyse für $C_{21}H_{25}N_5O_7S_2$ (523,55): | | | | |
|---|---|---|---|---|
| | C | H | N | S |
| Ber. | 48,17 | 4,81 | 13,38 | 12,25 |
| Gef. | 47,90 | 4,97 | 13,26 | 12,23 |

b) (E)-2-(t-Butoxycarbonyl)-2-butenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat als farblosen Festkorper.

| Elementaranalyse für $C_{23}H_{29}N_5O_7S_2$ (551,61): | | | | |
|---|---|---|---|---|
| | C | H | N | S |
| Ber. | 50,08 | 5,30 | 12,70 | 11,62 |
| Gef. | 49,89 | 5,39 | 12,60 | 12,69 |

c) (E)-2-(Isobutoxycarbonyl)-2-butenyl (6R,7R)-7-[(Z)-2-(2-amino-4-thiazolyl)-2-(methoxyimino)-acetamido]-3 methyl-8-oxo-5-thia-1-azabicyclo[4.2.0]oct-2-en-2-carboxylat als farblosen Festkörper (aus Aether/Methylenchlorid). Massenspektrum: protoniertes Molekülion bei m/e 552.

d) (E)-2-[(2-Aethoxyäthoxy)carbonyl]-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als farblosen Festkörper.

| Elementaranalyse für $C_{23}H_{29}N_5O_8S_2$ (567,60): | | | | |
|---|---|---|---|---|
| | C | H | N | S |
| Ber. | 48,67 | 5,15 | 12,34 | 11,30 |
| Gef. | 48,37 | 5,41 | 12,57 | 13,31 |

e) (E)-2-[(RS)-2-Aethylhexyloxycarbonyl]butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als Festkörper. Massenspektrum: u.a. Spitzen bei m/e 368.

f) (E)-2-[[(RS)-2,3-Dimethoxypropoxy]carbonyl]-2-butenyl (6R,7R)-7-[(Z)-2-(2-amino-4-thiazolyl)-2-(methoxyimino)acetamido]-8-oxo-5-thia-1-azabicyclo[4.2.0]oct-2-en-carboxylat als farblosen Festkörper.

| Elementaranalyse für $C_{24}H_{31}N_5O_9S_2$ (597,65): | | | | |
|---|---|---|---|---|
| | C | H | N | S |
| Ber. | 48,23 | 5,23 | 11,72 | 10,73 |
| Gef. | 48,00 | 5,40 | 11,40 | 10,38 |

g) (E)-2-Acetyl-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als gelblichen Festkörper (aus Aether/Methylenchlorid).

| Elementaranalyse für $C_{20}H_{23}N_5O_6S_2$ (493,53): | | | |
|---|---|---|---|
| | C | H | N |
| Ber. | 48,67 | 4,70 | 14,19 |
| Gef. | 48,80 | 5,23 | 13,16 |

h) (RS)-2-(Methoxycarbonyl)-2-cyclohexen-1-yl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als farblosen Festkörper (aus Methylenchlorid/ Aether).

| Elementaranalyse für $C_{22}H_{25}N_5O_7S_2$ (535,60): | | | |
|---|---|---|---|
| | C | H | N |
| Ber. | 49,34 | 4,71 | 13,08 |
| Gef. | 49,35 | 4,90 | 12,67 |

i) 1-Aethyl 4-methyl 2-[[[(6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-yl]carbonyl]methoxy]fumarat als farblosen Festkörper (aus Methylenchlorid/Aether).
Massenspektrum: protoniertes Molekülion bei m/e 568.

j) 1-Aethyl 4-methyl 2-[[[[(6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-yl]carbonyl]oxy]methyl]maleat als farblosen Festkörper (aus Methylenchlorid/Aether).
Massenspektrum: protoniertes Molekülion bei m/e 568.

k) (E)-2-(Aethoxycarbonyl)-2-pentenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat als farblosen Festkörper (aus Aether).

| Elementaranalyse für $C_{22}H_{27}N_5O_7S_2$ (537,58): | | | | |
|---|---|---|---|---|
| | C | H | N | S |
| Ber. | 49,15 | 5,06 | 13,03 | 11,93 |
| Gef. | 48,66 | 5,15 | 12,82 | 11,60 |

l) 2-(p-Nitrophenoxycarbonyl)allyl (6R,7R)-7-[(Z)-2-(2-amino-4-thiazolyl)-2-(methoxyimino)acetamido]-3-methyl-8-oxo-5-thia-1 azabicyclo[4.2.0]oct-2-en-2-carboxylat als gelben Festkörper.
IR-Spektrum (KBr): u.a. Banden bei 1778, 1739, 1681 und 1615 $cm^{-1}$.

m) (E)-2-[[(Tetrahydro-2H-pyran-4-yl)oxy]carbonyl]-2-hexenyl (6R,7R)-7-[(Z)-2-(2-amino-4-thiazolyl)-2-(methoxyimino)acetamido]-3-methyl-8-oxo-5-thia-1 azabicyclo[4.2.0]oct-2-en-2- carboxylat als farblosen Festkörper (aus Methylenchlorid/Hexan).
Massenspektrum: protoniertes Molekülion bei m/e 608.

n) (E)-2-[[(Tetrahydro-2H-pyran-4-yl)oxy]carbonyl]-2-pentenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als farblosen Festkörper (aus Methylenchlorid/Hexan).
IR-Spektrum (KBr): u.a. Banden bei 1781, 1715, 1683, 1619 und 1534 $cm^{-1}$.

o) (E)-2-[[(Tetrahydro-2H-pyran-4-yl)oxy]carbonyl]-2-butenyl (6R,7R)-7-[(Z)-2-(2-amino-4-thiazolyl)-2-(methoxyimino)acetamido]-8-oxo-5-thia-1-azabicyclo[4.2.0]oct-2-en-2-carboxylat als farblosen Festkörper (aus Essigester/Hexan).
Massenspektrum: protoniertes Molekülion bei m/e 580.

p) (E)-2-(Isobutoxycarbonyl)-2-pentenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat als farblosen Festkörper.
Massenspektrum: protoniertes Molekülion bei m/e 566.

q) (E)-2-[[(RS)-(Tetrahydro-2H-pyran-2-yl)methoxy]carbonyl]-2-hexenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als farblosen Festkörper.
Massenspektrum: protoniertes Molekülion bei m/e 622.

r) (E)-2-[[[(RS)-Tetrahydro-2H-pyran-2-yl]methoxy]carbonyl]-2-butenyl (6R,7R)-7-[(Z)-2-(2-amino-4-thiazolyl)-2-(methoxyimino)acetamido]-3-methyl-8-oxo-5 thia-1-azabicyclo[4.2.0]oct-2-en-2-carboxylat als farblosen Festkörper (aus Methylenchlorid/Hexan).
Massenspektrum: protoniertes Molekülion bei m/e 594.

s) (E)-2-[[[(RS)-Tetrahydro-2H-pyran 2-yl]methoxy]carbonyl]-2-pentenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als farblosen Festkörper (aus Hexan).
IR-Spektrum (KBr): u.a. Banden bei 1781, 1718, 1662 und 1620 $cm^{-1}$.

t) (E)-2-[(Cyclohexyloxy)carbonyl]-2-butenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat als farblosen Festkörper (aus Aether/Hexan).

| Elementaranalyse für $C_{25}H_{31}N_5O_7S_2$ (577,64): | | | |
|---|---|---|---|
| | C | H | N |
| Ber. | 51,98 | 5,41 | 12,12 |
| Gef. | 51,95 | 5,96 | 12,04 |

u) (E)-2-(Isopropoxycarbonyl]-2-butenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat als farblosen Festkörper (aus Aether/Hexan).
Massenspektrum: protoniertes Molekülion bei m/e 538.

v) (E)-2-[(Cyclohexylmethoxy)carbonyl]-2-butenyl (6R,7R)-7-[(Z)-2-(2-amino-4 thiazolyl)-2-(methoxyimino)acetamido]-3-methyl-8-oxo-5-thia-1-azabicyclo[4.2.0]oct-2-en-2-carboxylat als farblosen Festkörper (aus Essigester/Hexan).
Massenspektrum: protoniertes Molekülion bei m/e 592.

w) (E)-2-[[(3-Methyl-2-butenyl)oxy]carbonyl]-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als farblosen Festkörper (aus Aether/Pentan).
Massenspektrum: protoniertes Molekülion bei m/e 564.

x) (E)-2-[[[(E/Z)-3,7-Dimethyl-2,6-octadienyl]oxy]carbonyl]-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct- 2-en-2-carboxylat als farblosen Festkörper (aus Aether/Hexan).
Massenspektrum: protoniertes Molekülion bei m/e 632.

y) (E)-3-(5-Brom-2-furyl)-2-(äthoxycarbonyl)allyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als farblosen Festkörper (aus Aether/Hexan).

| Elementaranalyse für $C_{24}H_{24}BrN_5O_8S_2$ (654,47): | | | | | |
|---|---|---|---|---|---|
| | C | H | N | S | Br |
| Ber. | 44,04 | 3,70 | 10,70 | 9,80 | 12,21 |
| Gef. | 44,31 | 3,98 | 10,54 | 9,74 | 12,09 |

z) 2-(Aethoxycarbonyl)-2,4-hexadienyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat als farblosen Festkörper.

| Elementaranalyse für $C_{23}H_{27}N_5O_7S_2$ (549,61): | | | |
|---|---|---|---|
| | C | H | N |
| Ber. | 50,26 | 4,95 | 12,74 |
| Gef. | 49,95 | 5,33 | 12,10 |

aa) 2-(Dimethoxyphosphinyl)allyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als farblosen Festkörper (aus Aether/Hexan).
Massenspektrum: protoniertes Molekülion bei m/e 546.

ab) (E und/oder Z)-4,4,4-Trichlor-2-(äthoxycarbonyl)-2-butenyl (6R,7R)-7-[(Z)-2-(2-amino-4-thiazolyl)-2-(methoxyimino)acetamido]-3-methyl-8-oxo-5 thia-1-azabicyclo[4.2.0]oct-2-en-2-carboxylat als farblosen Festkörper.
Massenspektrum: Molekülion bei m/e 626.

ac) 2-(Piperidinocarbonyl)allyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als farblosen Fest körper (aus Essigester).
IR-Spektrum (KBr): u.a. Banden bei 1779, 1724, 1677, 1610, 1535 und 1452 $cm^{-1}$.

ad) (E)-2-(Aethoxycarbonyl)-2-butenyl (6R,7R)-7-[(Z)-2-(2-amino-4-thiazolyl)-2-(methoxyimino)-acetamido]-3-(azidomethyl)-8-oxo-5-thia-1-azabicyclo[4.2.0]oct-2-en-2-carboxylat als farblosen Festkörper.

| Elementaranalyse für $C_{21}H_{24}N_8O_7S_2$ (564,57): | | | | |
|---|---|---|---|---|
| | C | H | N | S |
| Ber. | 44,67 | 4,28 | 19,85 | 11,36 |
| Gef. | 44,83 | 4,31 | 19,58 | 11,27 |

ae) (E)-2-[(Cyclohexylmethoxy)carbonyl]-2-butenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat als farblosen Festkörper (aus Aether/Hexan).
IR-Spektrum (KBr); u.a. Banden bei 2104, 1788, 1717, 1618 und 1532 $cm^{-1}$.

Durch Behandeln dieser Verbindung mit 4,9N-Salzsäure in Isopropanol erhält man (E)-2-[(Cyclohexylmethoxy)carbonyl]-2-butenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat hydrochlorid als farblosen Festkörper (aus i-Propanol/Hexan).

| Elementaranalyse für $C_{26}H_{32}N_8O_7S_2$,1:1 HCl (669,17): | | | | | |
|---|---|---|---|---|---|
| | C | H | N | Cl | S |
| Ber. | 46,67 | 4,97 | 16,75 | 5,30 | 9,58 |
| Gef. | 46,28 | 5,10 | 16,54 | 5,20 | 9,39 |

af) (E)-2-(Isobutoxycarbonyl)-2-pentenyl (6R,7R)-7-[(Z)-2-(2-amino-4-thiazolyl)-2-(methoxyimino)-acetamido]-3-(azidomethyl)-8-oxo-5-thia-1-azabicyclo[4.2.0]oct-2-en-2-carboxylat als gelblichen Festkörper (aus Aether/Hexan).
Massenspektrum: protoniertes Molekülion bei m/e 607.

ag) (E)-2-(Isobutoxycarbonyl)-2-butenyl (6R,7R)-7-[(Z)-2-(2-amino-4-thiazolyl)-2-(methoxyimino)-acetamido]-3-(azidomethyl)-8-oxo-5-thia-1-azabicyclo[4.2.0]oct-2-en-2-carboxylat als farblosen Festkörper.
Massenspektrum: protoniertes Molekülion bei m/e 593.

ah) (E)-2-[(2-Aethoxyäthoxy)carbonyl]-2-butenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat als farblosen Festkörper.
Massenspektrum: protoniertes Molekülion bei m/e 609.

ai) (E)-2-(Aethoxycarbonyl)-2-butenyl (6R,7R)-3-(acetoxymethyl)-8-oxo-7-[2-(3-thienyl)acetamido]-5-thia-1-azabicyclo[4.2.0]oct-2-en-2-carboxylat als farblosen Festkörper mit einem Smp. von 88° (aus Aether/Hexan).

| Elementaranalyse für $C_{23}H_{26}N_2O_8S_2$ (522,56): | | | |
|---|---|---|---|
| | C | H | N |
| Ber. | 52,86 | 5,02 | 5,36 |
| Gef. | 52,45 | 4,85 | 5,44 |

aj) (E)-2-(Isobutoxycarbonyl)-2-pentenyl (6R,7R)-3-(acetoxymethyl)-7-[(Z)-2-(2-amino-4-thiazolyl)-2-(methoxyimino)acetamido]-8-oxo-5-thia-1-azabicyclo[4.2.0]oct-2-en-2-carboxylat.

| Elementaranalyse für $C_{26}H_{33}N_5O_9S_2$ (623,70): | | | |
|---|---|---|---|
| | C | H | N |
| Ber. | 50,07 | 5,33 | 11,23 |
| Gef. | 50,03 | 5,66 | 11,20 |

ak) (E)-2-(Isobutoxycarbonyl)-2-pentenyl (6R,7R)-7-[(Z)-2-(2-amino-4-thiazolyl)-2-(methoxyimino)-acetamido]-8-oxo-5-thia-1-azabicyclo[4.2.0]oct-2-en-2-carboxylat als farblosen Festkörper (aus Methylenchlorid/Aether).

| Elementaranalyse für $C_{23}H_{29}N_5O_7S_2$ (551,63): | | | |
|---|---|---|---|
| | C | H | N |
| Ber. | 50,08 | 5,30 | 12,70 |
| Gef. | 49,85 | 5,41 | 12,46 |

al) (E)-2-(Isobutoxycarbonyl)-2-pentenyl (6R,7R)-3-[(carbamoyloxy)methyl]-7-[(Z)-2-(2-furyl)-2-(methoxyimino)acetamido]-8-oxo-5-thia-1-azabicyclo[4.2.0]oct-2-en-2-carboxylat als farblosen Festkörper (aus Methylenchlorid/Aether).

| Elementaranalyse für $C_{26}H_{32}N_4O_{10}S$ (592,62): | | | |
|---|---|---|---|
| | C | H | N |
| Ber. | 52,70 | 5,44 | 9,45 |
| Gef. | 52,67 | 5,59 | 9,23 |

## Beispiel 4

1 mMol einer antibiotisch wirksamen Carbonsäure aus dem Gebiet der β-Lactam-Antibiotika, 1 mMol Triphenylphosphin und 1,5 mMol des gewünschten Alkohols der Formel II werden in 5 ml Dimethylacetamid gelöst. Die erhaltene Lösung wird tropfenweise mit einer Lösung von 1,03 nMol Diäthylazodicarboxylat in Dimethylacetamid behandelt. Man rührt während 4 Stunden, verdünnt das Reaktionsgemisch mit 100 ml Essigester und wäscht sie dreimal mit je 50 ml Wasser und zweimal mit je 50 ml 10-proz. wässriger Kochsalzlösung. Die organische Phase wird über Magnesiumsulfat getrocknet, filtriert und eingedampft. Der Rückstand wird dann durch Chromatographie an Kieselgel unter Eluieren mit Essigester/Hexan (4:1) gereinigt.

Die nachfolgend aufgeführten Verbindungen wurden auf diese Weise hergestellt:

a) (RS)-2-(Aethoxycarbonyl)-1,3-dimethyl-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat als farblosen Festkörper.
Massenspektrum; protoniertes Molekülion bei m/e 552.

b) (RS)-2-(Aethoxycarbonyl)-1-methylallyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat als farblosen Festkörper (aus Aether/Hexan).
Massenspektrum: protoniertes Molekülion bei m/e 524.

## Beispiel 5

a) 0,51 g (6R,7R)-7-Amino-3-azidomethyl-8-oxo-5-thia-1-azabicyclo[4.2.0]oct-2-en-2-carbonsäure werden in 50 ml Aceton gelöst, worauf man auf -10° abkühlt und mit 0.29 ml DBU versetzt. Nach 15 Minuten wird diese Lösung mit 0,54 g Isobutyl (Z)-2-(brommethyl)-2-pentenoat versetzt und über Nacht bei 0° gerührt. Die Lösung wird dann mit 50 ml Essigsäurebutylester verdünnt, und die Kristalle werden abgenutscht. Das Filtrat wird mit 30 ml Kochsalzlösung versetzt, und das Gemisch wird genutscht. Die organische Phase wird abgetrennt, getrocknet, filtriert und eingedampft. Der Rückstand wid an einer kurzen Kieselgelsäule unter Eluieren mit Essigester/Hexan (6:4) rasch chromatographiert. Die relevanten Fraktionen werden vereinigt und eingedampft. Der Rückstand wird in 20 ml Essigester aufgenommen und mit 4N Salzsäure in Essigester versetzt. Nach 24 Stunden bei 0° werden die Kristalle abfiltriert und unter vermindertem Druck getrocknet. Man erhält 0,38 g (E)-2-(Isobutoxycarbonyl)-2-pentenyl (6R,7R)-7-amino-3-azidomethyl-8-oxo-5-thia-1-azabicyclo[4.2.0]oct-2-en-carboxylat Hydrochlorid.

| Elementaranalyse für $C_{18}H_{26}N_5O_5Cl$ (459,95): | | | |
|---|---|---|---|
| | C | H | N |
| Ber. | 47,00 | 5,70 | 15,23 |
| Gef. | 46,87 | 5,78 | 15,14 |

b) 0,32 g (E)-2-(Isobutoxycarbonyl)-2-pentenyl (6R,7R)-7-amino-3-azidomethyl-8-oxo-5-thia-1-azabicyclo[4.2.0]oct-2-en-2-carboxylat-Hydrochlorid werden in 15 ml Methylenchlorid gelöst, worauf man mit 0,24 g S-(2-Benzothiazolyl)-2-amino-4-thiazolglyoxylat (Z)-O-methyloxim versetzt. Nach 6 Stunden wird das Reaktionsgemisch eingedampft, und der Rückstand wird an einer kurzen Kieselgelsäule unter Eluieren mit Essigester/Hexan (1:1) und dann mit Essigester rasch chromatographiert. Die relevanten Fraktionen werden vereinigt und eingedampft. Der Rückstand wird in 20 ml Essigester gelöst, worauf man mit 5 ml 4N Salzsäure in Essigester versetzt. Die erhaltenen Kristalle werden abgenutscht und aus Isopropanol umkristallisiert. Man erhält 0,2 g (E)-2-(Isobutoxycarbonyl)-2-pentenyl (6R,7R)-7-[(Z)-2-(2-amino-4-thiazolyl)-2-(methoxyimino)acetamido]-3-azidomethyl-8-oxo-5-thia-1-azabicyclo[4.2.0]oct-2-en-2-carboxylat.

| Elementaranalyse für $C_{24}H_{30}ClN_8O_7S_2$ (693,13): | | | |
|---|---|---|---|
| | C | H | N |
| Ber. | 44,82 | 4,86 | 17,42 |
| Gef. | 43,41 | 4,72 | 16,02 |

Beispiel A

Es wird in üblicher Weise eine Gelatine-Steckkapsel folgender Zusammensetzung hergestellt:

| | |
|---|---|
| (E)-2-(Isobutoxycarbonyl)-2-pentenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat | 500 mg |
| Luviskol (wasserlösliches Polyvinylpyrrolidon) | 20 mg |
| Mannit | 20 mg |
| Talk | 15 mg |
| Magnesiumstearat | 2 mg |
| Kapselfüllgewicht | 557 mg |

Beispiel B

Es wird in üblicher Weise eine Tablette folgender Zusammensetzung hergestellt:

| | |
|---|---|
| (E)-2-(Isobutoxycarbonyl)-2-pentenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat | 250 mg |
| Lactose | 70 mg |
| Maisstärke | 65 mg |
| Polyvinylpyrrolidon | 10 mg |
| Magnesiumstearat | 5 mg |
| Tablettengewicht | 400 mg |

Mit dem Wirkstoff, der Lactose, dem Polyvinylpyrrolidon und 40 Gewichtsteilen der Maisstärke wird in üblicher Weise ein Granulat hergestellt. Dieses wird mit den restlichen 25 Gewichtsteilen Maisstärke und 5 Gewichtsteilen Magnesiumstearat vermischt und zu Tabletten verpresst.

## Ansprüche

1. Unter physiologischen Bedingungen spaltbare Ester von pharmakologisch wirksamen Carbonsäuren, insbesondere von antibiotisch wirksamen Carbonsäuren aus dem Gebiet der $\beta$-Lactam-Antibiotika, dadurch gekennzeichnet, dass die Alkohol-Komponente des Esters eine Gruppe der allgemeinen Formel

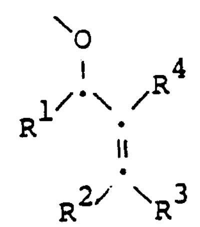

i

ist,
worin $R^1$ Wasserstoff oder niederes Alkyl, $R^2$ Wasserstoff, niederes Alkyl, niederes Halogenalkyl, niederes Alkenyl, niederes Alkoxycarbonyl, Aryl oder Heteroaryl oder zusammen mit $R^1$ niederes Alkylen, $R^3$ Wasserstoff, niederes Alkyl oder niederes Alkoxycarbonyl, $R^4$ die Gruppe $-COOR^5$, $-COR^6$, $-SO_2-R^6$, $-CONR^7R^8$ oder $-PO(OR^9)_2$, $R^5$ einen gesättigten oder ungesättigten Kohlenwasserstoffrest mit bis zu 12 Kohlenstoffatomen, in welchem bis zu zwei Methylengruppen durch Sauerstoffatome ersetzt sein können, Aryl oder Aryl-niederes Alkyl, $R^6$ niederes Alkyl oder Aryl, $R^7$ und $R^8$ je Wasserstoff oder niederes Alkyl oder zusammen niederes Alkylen, in welchem eine Methylengruppe durch ein Sauerstoff- oder Schwefelatom oder durch eine Imino- oder niedere Alkyliminogruppe ersetzt sein kann, und $R^9$ niederes Alkyl bedeuten,
und, sofern ein basischer Substituent vorhanden ist, pharmazeutisch annehmbare Säureadditionssalze davon.

2. Ester nach Anspruch 1, dadurch gekennzeichnet, dass die Carbonsäure Komponente ein pharmazeutisch annehmbarer Penam-3-carbonsäure-, Penem-3-carbonsäure-, 1-Carbapenem-3-carbonsäure-, 3-Cephem-4-carbonsäure-, 1-Oxa-3-cephem- 4-carbonsäure-, 1-Dethia-2-oxa-3-cephem-, 1-Dethia-2-thia-3-cephem- oder ein 1-Carba-3-cephem-4-carbonsäurerest ist.

3. Ester nach Anspruch 2, dadurch gekennzeichnet, dass die Carbonsäure Komponente ein pharmazeutisch annehmbarer 3-Cephem-4-carbonsäurerest ist.

4. Ester nach Anspruch 3 der allgemeinen Formel

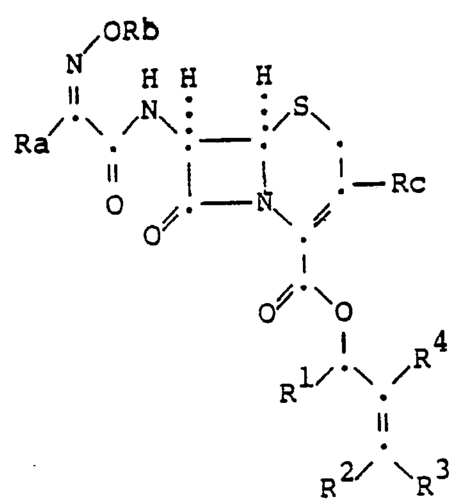

I

worin Ra Aryl oder Heteroaryl, Rb Wasserstoff, niederes Alkanoyl oder gegebenenfalls durch Carboxy,

Halogen, $C_{3-6}$-Cyloalkyl oder Heteroaryl substituiertes niederes Alkyl oder $C_{3-6}$-Cycloalkyl, Rc Wasserstoff, Halogen, niederes Alkyl, niederes Alkenyl, niederes Alkoxy oder die Gruppe $-CH_2$-Rd oder $-CH_2$-S-Re, Rd niederes Alkanoyloxy, Carbamoyloxy, Azido oder eine über ein Stickstoffatom gebundene, N-haltige aromatische Gruppe und Re eine über ein Kohlenstoffatom gebundene heteroaromatische Gruppe bedeuten, und $R^1$, $R^2$ $R^3$ und $R^4$ die in Anspruch 1 angegebene Bedeutung besitzen.

5. Ester nach Anspruch 4, worin Ra eine unsubstituierte oder durch Amino substituierte 5-gliedrige heteroaromatische Gruppe mit einem Schwefel- oder Sauerstoffatom und gegebenenfalls noch einem oder zwei Stickstoffatomen als Heteroringglieder bedeutet.

6. Ester nach Anspruch 5, worin Ra 2-Amino-4-thiazolyl, 5-Amino-1,2,4-thiadiazol-3-yl oder 2-Furyl, insbesondere 2-Amino-4-thiazolyl bedeutet.

7. Ester nach einem der Ansprüche 4-6, worin Rb Wasserstoff, niederes Alkyl oder niederes Alkanoyl, insbesondere niederes Alkyl bedeutet.

8. Ester nach einem der Ansprüche 4-7, worin Rc niederes Alkyl, insbesondere Methyl, oder die Gruppe $-CH_2$-Rd und Rd Azido bedeuten.

9. Ester nach einem der Ansprüche 1-8, worin $R^1$ Wasserstoff bedeutet.

10. Ester nach einem der Ansprüche 1-9, worin $R^2$ niederes Alkyl bedeutet.

11. Ester nach einem der Ansprüche 1-10, worin $R^3$ Wasserstoff bedeutet.

12. Ester nach einem der Ansprüche 1-11, worin $R^4$ die Gruppe $-COOR^5$ und $R^5$ niederes Alkyl, niederes Alkenyl oder niederes Alkoxyalkyl bedeuten.

13. Ester nach Anspruch 12, worin $R^5$ niederes Alkyl oder niederes Alkoxyalkyl, insbesondere Isobutyl, Isopropyl oder 2-Methoxyäthyl, vorzugsweise Isobutyl bedeutet.

14. (E)-2-(Isobutoxycarbonyl)-2-pentenyl (6R,7R)-7-[(Z)-2-(2-amino-4-thiazolyl)-2-(methoxyimino)-acetamido]-3-(azidomethyl)-8-oxo-5-thia-1-azabicyclo[4.2.0]oct-2-en-2-carboxylat.

15. (E)-2-(Isobutoxycarbonyl)-2-butenyl (6R,7R)-7-[(Z)-2-(2-amino-4 thiazolyl)-2-(methoxyimino)-acetamido]-3-(azido methyl)-8-oxo-5-thia-1-azabicyclo[4.2.0]oct-2-en-2-carboxylat.

16. (E)-2-[(2-Aethoxyäthoxy)carbonyl]-2-butenyl (6R,7R)-7-[(Z)-2-(2-amino-4-thiazolyl)-2-(methoxyimino)-acetamido]-3-(azidomethyl)-8-oxo-5-thia-1-azabicyclo[4.2.0]oct-2-en-2-carboxylat.

17. (E)-2-(Aethoxycarbonyl)-2-butenyl (6R,7R)-7-[(Z)-2-(2-amino-4-thiazolyl)-2-(methoxyimino)-acetamido]-3-(azidomethyl)-8-oxo-5-thia-1-azabicyclo[4.2.0]oct-2-en-2-carboxylat.

18. (E)-2-[[(3-Methyl-2-butenyl)oxy]carbonyl]-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat.

19. (E)-2-(Isopropoxycarbonyl)-2-butenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat.

20. (E)-2-(Isobutoxycarbonyl)-2-pentenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat.

21. (E)-2-(Aethoxycarbonyl)-2-pentenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat.

22. (E)-2-[(2-Aethoxyäthoxy)carbonyl]-2-butenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat.

23. (E)-2-(Isobutoxycarbonyl)-2-butenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat.

24. · (E)-2-(t-Butoxycarbonyl)-2-butenyl (6R,7R)-7-[(Z)-2-(2-amino-4-thiazolyl)-2-(methoxyimino)-acetamido]-3-methyl-8-oxo-5-thia-1-azabicyclo[4.2.0]oct-2-en-2-carboxylat.

25. (E)-2-(Aethoxycarbonyl)-2-butenyl (6R,7R)-7-[(Z)-2-(2-amino-4-thiazolyl)-2-(methoxyimino)-acetamido]-3-methyl-8-oxo-5-thia-azabicyclo[4.2.0]oct-2-en-2-carboxylat.

26. Verbindungen der allgemeinen Formel

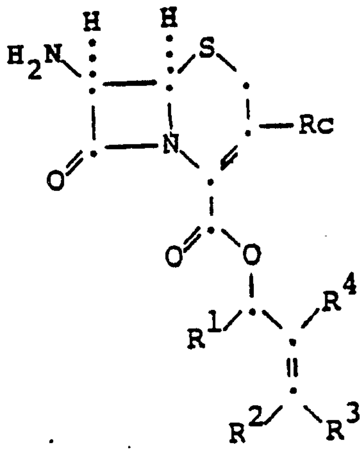

III

worin $R^1$, $R^2$, $R^3$ und $R^4$ die in Anspruch 1 und Rc die in Anspruch 4 angegebene Bedeutung besitzen.

27. Ester nach einem der Ansprüche 1-25, zur Anwendung bei der Verhütung oder Bekämpfung von Krankheiten.

28. Ester nach einem der Ansprüche 1-25, zur Anwendung bei der Verhütung oder Bekämpfung von Infektionskrankheiten.

29. Ester nach einem der Ansprüche 1-25, zur peroralen Anwendung als antibiotisch wirksame Stoffe.

30. Verfahren zur Herstellung von Estern nach einem der Ansprüche 1-25 und von pharmazeutisch annehmbaren Säureadditionssalzen davon, dadurch gekennzeichnet, dass man

a) eine pharmakologisch wirksame Carbonsäure, insbesondere eine antibiotisch wirksame Carbonsäure aus dem Gebiet der $\beta$-Lactam-Antibiotika mit einer Verbindung der allgemeinen Formel

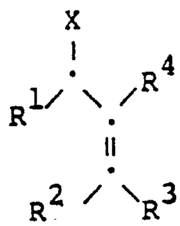

II

worin X Hydroxy oder eine Abgangsgruppe bedeutet, und $R^1$, $R^2$, $R^3$ und $R^4$ die in Anspruch 1 angegebene Bedeutung haben,
verestert, oder

b) eine Verbindung der allgemeinen Formel

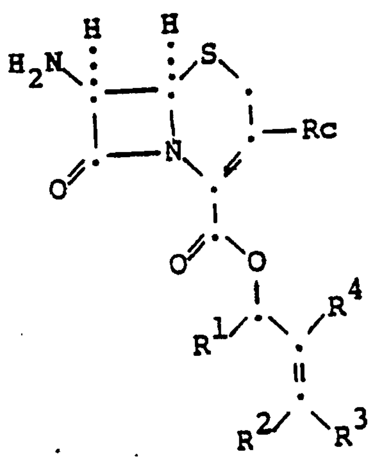

III

worin $R^1$, $R^2$, $R^3$ und $R^4$ die in Anspruch 1 und Rc die in Anspruch 4 angegebene Bedeutung besitzen. mit

einer Carbonsäure der allgemeinen Formel

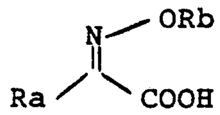

IV

worin Ra und Rb die in Anspruch 4 angegebene Bedeutung besitzen, oder einem reaktiven Derivat davon acyliert und

c) erwünschtenfalls den erhaltenen Ester in ein pharmazeutisch annehmbares Säureadditionssalz überführt.

31. Verfahren nach Anspruch 30, dadurch gekennzeichnet, dass man in der Verfahrensvariante a) die pharmakologisch wirksame Carbonsäure in Form eines Carbonsäuresalzes mit einer Verbindung der Formel II umsetzt, worin X eine Abgangsgruppe bedeutet.

32. Verfahren nach Anspruch 30, dadurch gekennzeichnet, dass man in der Verfahrensvariante a) die pharmakologisch wirksame Carbonsäure in Gegenwart eines Kondensationsmittels mit einer Verbindung der Formel II umsetzt, worin X eine Hydroxygruppe bedeutet.

33. Arzneimittel, enthaltend als Wirkstoff einen Ester nach einem der Ansprüche 1-25 und ein therapeutisch inertes Trägermaterial.

34. Arzneimittel zur peroralen Verabreichung, enthaltend als Wirkstoff einen Ester nach einem der Ansprüche 1-25 und ein für perorale Arzneimittel übliches, therapeutisch inertes Trägermaterial.

35. Antibiotisch wirksames Mittel, enthaltend als Wirkstoff einen Ester nach einem der Ansprüche 1-25 und ein therapeutisch inertes Trägermaterial.

36. Antibiotisch wirksames Mittel zur peroralen Verabreichung, enthaltend als Wirkstoff einen Ester nach einem der Ansprüche 1-25 und ein für perorale Arzneimittel übliches, therapeutisch inertes Trägermaterial.

37. Verwendung von Estern nach einem der Ansprüche 1-25 zur Verhütung oder Bekämpfung von Krankheiten.

38. Verwendung von Estern nach einem der Ansprüche 1-25 zur Verhütung oder Bekämpfung von Infektionskrankheiten.

39. Verwendung von Estern nach einem der Ansprüche 1-25 zur Herstellung von therapeutisch, insbesondere antibiotisch wirksamen Mitteln.

40. Verwendung von Estern nach einem der Ansprüche 1-25 zur Herstellung von peroral zu verabreichenden, therapeutisch, insbesondere antibiotisch wirksamen Mitteln.

41. Verwendung von Verbindungen der allgemeinen Formel

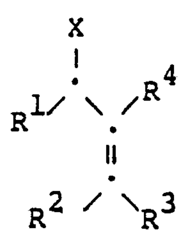

II

worin $R^1$, $R^2$, $R^3$ und $R^4$ die in Anspruch 1 und X die in Anspruch 30 angegebene Bedeutung besitzen, bei der Veresterung von therapeutisch wirksamen Carbonsäuren, insbesondere von antibiotisch wirksamen Carbonsäuren aus dem Gebiet der β-Lactam-Antibiotika.

Patentansprüche für folgende Vertragsstaaten : ES, GR

1. Verfahren zur Herstellung von unter physiologischen Bedingungen spaltbaren Estern von pharmakologisch wirksamen Carbonsäuren, insbesondere von antibiotisch wirksamen Carbonsäuren aus dem Gebiet der β-Lactam-Antibiotika, wobei die Alkohol-Komponente des Esters eine Gruppe der allgemeinen Formel

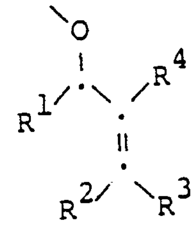

;

ist,

worin $R^1$ Wasserstoff oder niederes Alkyl, $R^2$ Wasserstoff, niederes Alkyl, niederes Halogenalkyl, niederes Alkenyl, niederes Alkoxycarbonyl, Aryl oder Heteroaryl oder zusammen mit $R^1$ niederes Alkylen, $R^3$ Wasserstoff, niederes Alkyl oder niederes Alkoxycarbonyl, $R^4$ die Gruppe $-COOR^5$, $-COR^6$, $-SO_2-R^6$, $-CONR^7R^8$ oder $-PO(OR^9)_2$, $R^5$ einen gesättigten oder ungesättigten Kohlenwasserstoffrest mit bis zu 12 Kohlenstoffatomen, in welchem bis zu zwei Methylengruppen durch Sauerstoffatome ersetzt sein können, Aryl oder Aryl-niederes Alkyl, $R^6$ niederes Alkyl oder Aryl, $R^7$ und $R^8$ je Wasserstoff oder niederes Alkyl oder zusammen niederes Alkylen, in welchem eine Methylengruppe durch ein Sauerstoff- oder Schwefelatom oder durch eine Imino- oder niedere Alkyliminogruppe ersetzt sein kann, und $R^9$ niederes Alkyl bedeuten,

und, sofern ein basischer Substituent vorhanden ist, von pharmazeutisch annehmbaren Säureadditionssalzen davon, dadurch gekennzeichnet, dass man

a) eine pharmakologisch wirksame Carbonsäure, insbesondere eine antibiotisch wirksame Carbonsäure aus dem Gebiet der $\beta$-Lactam-Antibiotika mit einer Verbindung der allgemeinen Formel

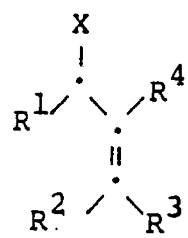

II

worin X Hydroxy oder eine Abgangsgruppe bedeutet, und $R^1$, $R^2$, $R^3$ und $R^4$ obige Bedeutung haben, verestert, oder

b) zur Herstellung eines Esters der allgemeinen Formel

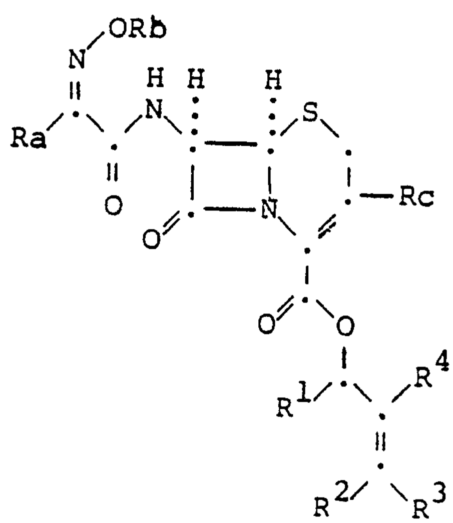

I

worin Ra Aryl oder Heteroaryl, Rb Wasserstoff niederes Alkanoyl oder gegebenenfalls durch Carboxy, Halogen, $C_{3-6}$-Cyloalkyl oder Heteroaryl substituiertes niederes Alkyl oder $C_{3-6}$-Cycloalkyl, Rc Wasserstoff, Halogen, niederes Alkyl, niederes Alkenyl, niederes Alkoxy oder die Gruppe $-CH_2$-Rd oder $-CH_2$-S-Re,

Rd niederes Alkanoyloxy, Carbamoyloxy, Azido oder eine über ein Stickstoffatom gebundene, N-haltige aromatische Gruppe und Re eine über ein Kohlenstoffatom gebundene heteroaromatische Gruppe bedeuten, und $R^1$, $R^2$, $R^3$ und $R^4$ obige Bedeutung besitzen,
eine Verbindung der allgemeinen Formel

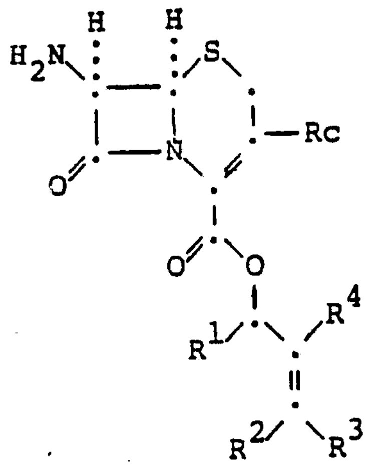

III

worin $R^1$, $R^2$, $R^3$, $R^4$ und Rc obige Bedeutung besitzen,
mit einer Carbonsäure der allgemeinen Formel

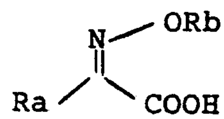

IV

worin Ra und Rb obige Bedeutung besitzen,
oder einem reaktiven Derivat davon acyliert und

c) erwünschtenfalls den erhaltenen Ester in ein pharmazeutisch annehmbares Säureadditionssalz überführt.

2. Verfahren nach Anspruch 1, dadurch gekennzeichnet, dass man in der Verfahrensvariante a) die pharmakologisch wirksame Carbonsäure in Form eines Carbonsäuresalzes mit einer Verbindung der Formel II umsetzt, worin X eine Abgangsgruppe bedeutet.

3. Verfahren nach Anspruch 1, dadurch gekennzeichnet, dass man in der Verfahrensvariante a) die pharmakologisch wirksame Carbonsäure in Gegenwart eines Kondensationsmittels mit einer Verbindung der Formel II umsetzt, worin X eine Hydroxygruppe bedeutet.

4. Verfahren nach einem der Ansprüche 1-3, dadurch gekennzeichnet, dass man einen Ester herstellt, in welchem die Carbonsäure-Komponente ein pharmazeutisch annehmbarer Penam-3-carbonsäure-, Penem-3-carbonsäure-, 1-Carbapenem-3-carbonsäure-, 3-Cephem-4-carbonsäure-, 1-Oxa-3-cephem-4-carbonsäure-, 1-Dethia-2-oxa-3 cephem-, 1-Dethia-2-thia-3-cephem- oder ein 1-Carba 3-cephem 4-carbonsäurerest ist.

5. Verfahren nach Anspruch 4, dadurch gekennzeichnet, dass die Carbonsäure-Komponente ein pharmazeutisch annehmbarer 3-Cephem-4-carbonsäurerest ist.

6. Vefahren nach einem der Ansprüche 1-3, dadurch gekennzeichnet, dass man einen Ester der allgemeinen Formel

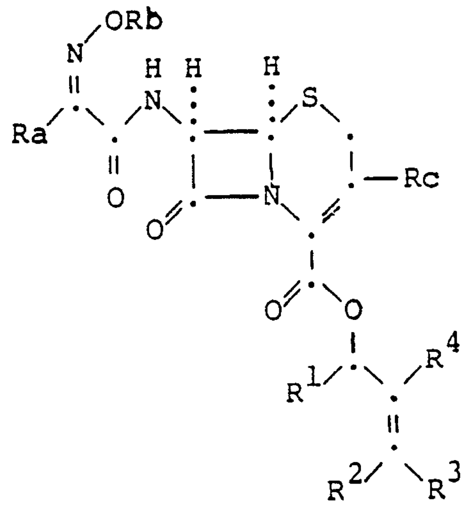

I

worin Ra, Rb, Rc, $R^1$, $R^2$, $R^3$ und $R^4$ die in Anspruch 1 angegebene Bedeutung besitzen, herstellt.

7. Verfahren nach Anspruch 6, worin Ra eine unsubstituierte oder durch Amino substituierte 5-gliedrige heteroaromatische Gruppe mit einem Schwefel- oder Sauerstoffatom und gegebenenfalls noch einem oder zwei Stickstoffatomen als Heteroringglieder bedeutet.

8. Verfahren nach Anspruch 7, worin Ra 2-Amino-4-thiazolyl, 5-Amino-1,2,4-thiadiazol-3-yl oder 2-Furyl, insbesondere 2-Amino-4-thiazolyl bedeutet.

9. Verfahren nach einem der Ansprüche 6-8, worin Rb Wasserstoff, niederes Alkyl oder niederes Alkanoyl, insbesondere niederes Alkyl bedeutet.

10. Verfahren nach einem der Ansprüche 6-9, worin Rc niederes Alkyl, insbesondere Methyl, oder die Gruppe $-CH_2$-Rd und Rd Azido bedeuten.

11. Verfahren nach einem der Ansprüche 1-10, worin $R^1$ Wasserstoff bedeutet.

12. Verfahren nach einem der Ansprüche 1-11, worin $R^2$ niederes Alkyl bedeutet.

13. Verfahren nach einem der Ansprüche 1-12, worin $R^3$ Wasserstoff bedeutet.

14. Verfahren nach einem der Ansprüche 1-13, worin $R^4$ die Gruppe $-COOR^5$ und $R^5$ niederes Alkyl, niederes Alkenyl oder niederes Alkoxyalkyl bedeuten.

15. Verfahren nach Anspruch 14, worin $R^5$ niederes Alkyl oder niederes Alkoxyalkyl, insbesondere Isobutyl, Isopropyl oder 2-Methoxyäthyl, vorzugsweise Isobutyl bedeutet.

16. Verfahren nach einem der Ansprüche 1-3, dadurch gekennzeichnet, dass man (E)-2-(Isobutoxycarbonyl)-2-pentenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat herstellt.

17. Verfahren nach einem der Ansprüche 1-3, dadurch gekennzeichnet, dass man (E)-2-(Isobutoxycarbonyl)-2-butenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat herstellt.

18. Verfahren nach einem der Ansprüche 1-3, dadurch gekennzeichnet, dass man (E)-2-[(2-Aethoxyäthoxy)carbonyl]-2-butenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat herstellt.

19. Verfahren nach einem der Ansprüche 1-3, dadurch gekennzeichnet, dass man (E)-2-(Aethoxycarbonyl)-2-butenyl (6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-en-2-carboxylat herstellt.

20. Verfahren nach einem der Ansprüche 1-3, dadurch gekennzeichnet, dass man (E)-2-[[(3-Methyl-2-butenyl)oxy]carbonyl]-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat herstellt.

21. Verfahren nach einem der Ansprüche 1-3, dadurch gekennzeichnet, dass man (E)-2-(Isopropoxycarbonyl)-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat herstellt.

22. Verfahren nach einem der Ansprüche 1-3, dadurch gekennzeichnet, dass man (E)-2-(Isobutoxycarbonyl)-2-pentenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat herstellt.

23. Verfahren nach einem der Ansprüche 1-3, dadurch gekennzeichnet, dass man (E)-2-(Aethoxycarbonyl)-2-pentenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat herstellt.

24. Verfahren nach einem der Ansprüche 1-3, dadurch gekennzeichnet, dass man (E)-2-[(2-Aethoxyäthoxy)carbonyl]-2-butenyl (6R,7R)-7-[(Z)-2-(2-amino 4-thiazolyl)-2-(methoxyimino)acetamido]-3-methyl-8-oxo-5-thia-1-azabicyclo[4.2.0]oct-2-en-2-carboxylat herstellt.

25. Verfahren nach einem der Ansprüche 1-3, dadurch gekennzeichnet, dass man (E)-2-(Isobutoxycarbonyl)-2-butenyl (6R,7R)-7-[(Z)-2-(2-amino-4-thiazolyl)-2-(methoxyimino)acetamido]-3-methyl-8-oxo-5 thia-1-azabicyclo[4.2.0]oct-2-en-2-carboxylat herstellt.

26. Verfahren nach einem der Ansprüche 1-3, dadurch gekennzeichnet, dass man (E)-2-(t-Butoxycarbonyl)-2-butenyl (6R,7R)-7-[(Z)-2-(2-amino-4-thiazolyl)-2-(methoxyimino)acetamido]-3-methyl-8-oxo-5-thia-1-azabicyclo[4.2.0]oct-2-en-2-carboxylat herstellt.

27. Verfahren nach einem der Ansprüche 1-3, dadurch gekennzeichnet, dass man (E)-2-(Aethoxycarbonyl)-2-butenyl (6R,7R)-7-[(Z)-2-(2-amino-4-thiazolyl)-2-(methoxyimino)acetamido]-3-methyl-8-oxo-5-thia-azabicyclo[4.2.0]oct-2-en-2-carboxylat herstellt.

28. Verfahren zur Herstellung von Arzneimitteln, insbesondere von antibiotisch wirksamen Mitteln, enthaltend als Wirkstoff einen gemäss den Ansprüchen 1-27 erhältlichen Estern, dadurch gekennzeichnet, dass man den Wirkstoff und gegebenenfalls eine andere therapeutisch wirksame Verbindung zusammen mit einem therapeutisch inerten Trägermaterial in eine galenische Darreichungsform bringt.

29. Verfahren zur Herstellung von Arzneimitteln zur peroralen Verabreichung, insbesonderevon antibiotisch wirksamen Mitteln, enthaltend als Wirkstoff einen gemäss den Ansprüchen 1-27 erhältlichen Ester, dadurch gekennzeichnet, dass man den Wirkstoff und gegebenenfalls eine andere therapeutisch wirksame Verbindung zusammen mit einem für perorale Arzneimittel üblichen therapeutisch inerten Trägermaterial in eine galenische Darreichungsform bringt.

30. Verwendung von Estern, welche nach einem der Ansprüche 1-27 erhältlich sind, zur Herstellung von therapeutisch, insbesondere antibiotisch wirksamen Mitteln.

31. Verwendung von Estern, welche nach einem der Ansprüche 1-27 erhältlich sind, zur Herstellung von peroral zu verabreichenden, therapeutisch, insbesondere antibiotisch wirksamen Mitteln.

32. Verwendung von Verbindungen der allgemeinen Formel

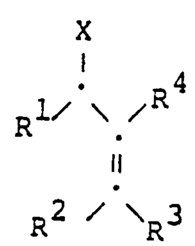

II

worin $R^1$, $R^2$, $R^3$, $R^4$ und X die in Anspruch 1 angegebene Bedeutung besitzen,
bei der Veresterung von therapeutisch wirksamen Carbonsäuren, insbesondere von antibiotisch wirksamen Carbonsäuren aus dem Gebiet der β-Lactam-Antibiotika.

33. Verbindungen der allgemeinen Formel

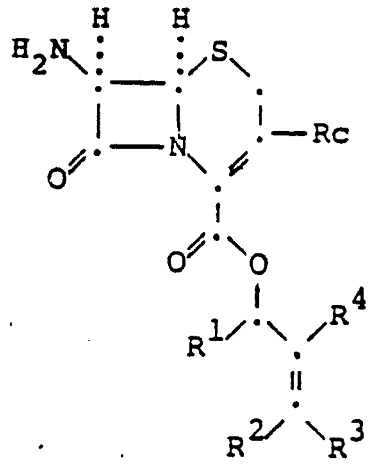

III

worin $R^1$, $R^2$, $R^3$, $R^4$ und Rc die in Anspruch 1 angegebene Bedeutung besitzen.